# EUROPEAN PATENT SPECIFICATION

(11) **EP 1 752 114 B1**
(45) Date of publication and mention of the grant of the patent: **04.11.2009**
(21) Application number: 06022771.7
(22) Date of filing: 21.09.2001
(51) Int. Cl.: A61F 2/90

(54) **Intravascular stent apparatus**
Intravaskulärer Stent
Extenseur intravasculaire

(30) Priority: 25.09.2000 US 235115 P
(43) Date of publication of application: 14.02.2007
(62) Divisional of application: 01973394.8
(73) Proprietor: Boston Scientific Scimed, Inc., Maple Grove, MN 55311 (US)
(72) Inventor: Jang, G. David, Redlands CA 92374 (US)
(74) Representative: Hauck Patent- und Rechtsanwälte

(56) References cited:
- EP-A- 0 875 215
- WO-A-97/25937
- WO-A-99/40876
- FR-A- 2 785 174
- US-A- 5 948 016

## Description

### BACKGROUND OF THE INVENTION

### Field of Invention:

This invention relates to intravascular stents in general, and more particularly to intracoronary stents.

### Description of the Related Art:

Intracoronary stents provide intraluminal scaffolding support of the vascular wall after percutaneous angioplasty in which the balloon catheter is used to expand the stenotic vascular lesion. In both the delivery phase and the deployed phase, there are numerous performance factors that can characterize the overall clinical performance of a stent and can be improved.

By the year 2000, the percutaneous balloon angioplasty and stent implant procedures have become the dominant non-surgical revascularization method of the atherosclerotic stenosis, or obstruction, of the vascular lumen, and particularly in the coronary vascular system of the heart. With balloon angioplasty alone and without stents, the restenosis rate after angioplasty has been as high as 25-45% in the first time coronary cases. With stents after balloon angioplasty, the restenosis rate has been reduced significantly. Even so, the restenosis rate after stent implantation is reported to be 15-25% range in coronary arteries, depending on the condition of the stented vessel or the specific stent. An ideal coronary stent is still elusive in the current state of the art commercial products.

Some of the best selling current, second generation, stents can be divided into two categories. One category is a stent with high flexibility and the other category has full vessel coverage. The flexible stents generally have poor vessel coverage, tissue prolapse, rough surface modulation and increased restenosis rate. On the other hand, a stent with good vessel coverage in the current state of art may not be flexible enough for easy delivery and for highly efficient procedures. This means that a stent with good flexibility and good vessel coverage remains as the unreached gold standard.

To further reduce the restenosis rate after stent implant, numerous means have been tried including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient, and costly. Brachytherapy is a radioactive device and a radiation therapy specialist from another department has to be involved with the interventional cardiologist in the cardiac catheterization laboratory. The laser and atherectomy devices proved to be marginally useful with added costs.

Local drug therapy appears to be a very promising method for the future, as better pharmaceutical, chemical, or biogenetic agents are developed and became available. Some research data, both from animal tests and human clinical studies, indicate evidence of some suppression of restenosis after stent implantation when certain growth blocking pharmaceutical agents coat the stent. In other instances, it has been speculated that certain surface modifying materials coated on the surface of the stent may be beneficial, alone or in combination with growth suppressing agents, in reducing the restenosis rate. In either instance, a drug or substance should be locally attached or coated on the stent in sufficient amounts. However, attaching or coating a sufficient amount of a substance or drug on the coronary stent may not be an easy proposition, because coating enough volume of the drug on the small surface area of a stent is a challenging task. If and when stent coating becomes practical, a good stent can still have better outcomes than a poorly designed stent when used with substance coating.

A stent is a scaffolding device. When delivered to a remote vessel location via percutaneous approach it can be deployed by expanding the device inside a vessel. The vessel can have a very small caliber and sometimes has a very tortuous anatomy. When a stent is deployed, the stent should have a good radial strength, a good vessel coverage, a good internal surface modulation without tulips (i.e., sharp metal loop projections that resemble fish scale phenomena), an optimal vessel conformability, a low metal fraction, and so forth. If the stent is stiff and non-flexible, it can be very difficult to deliver to an intended lesion site inside a vessel. Easy delivery of a stent is aided by good flexibility of the stent in combination with the delivery balloon, a smooth surface modulation without or minimizing tulips and a degree of radiopacity. A good stent should have a combination of features for delivery and deployment.

Although there are countless variations of vascular stent designs today, few have these desired stent features both in the delivery phase and in the post-delivery phase. Today's top selling stents in the market can have undesirable characteristics, either in the delivery phase or in the deployed phase of the stent life cycle. For example, some stents may have flexibility, but lack vessel coverage or surface modulations both in delivery and deployed phases. Some stents may have good vessel coverage and surface modulations, but lack flexibility.

Vascular stents, which are designed to be delivered to vessel sites via percutaneous approach, can have two elements. The first element is the expansion strut that expands circumferentially to provide the scaffolding radial force against a possible collapsing force of the vessel wall. The second element is the connecting strut that can link the expansion struts along the longitudinal axis of the stent, giving articulation or flexibility to the stent. The particular combination of expansion struts and connecting struts generally form various cells, depending on the specific configuration and shape of the expansion and connecting struts. If a cell is too large, the vessel wall support or coverage can be poor and the vessel wall tissue can prolapse through the large cells of the stent net. If the cells are too small, the vessel wall may be well covered but the metal fraction of the stent can be too high. The metal fraction is a fraction of the total metal surface area of an expanded stent (inside a blood vessel) divided by the total internal vessel wall surface area where the stent is deployed.

Some very flexible stents have very large cell size with poor vessel coverage and tissue prolapse, in addition to poor (inner and/or outer) surface modulation due to large numbers of tulips directed to both ends of the stent. Most of the current flexible stents are designed to effect flexibility by using fewer or a minimal number of connecting struts, handicapping the vessel coverage, surface modulation and tissue prolapse defects.

On the other hand, a stent that is designed for good vessel coverage and ideal cell size tends to be inflexible when such a stent is being delivered to a vessel lesion. A lack of flexibility during stent delivery is a very critical issue; a stiff stent often cannot be delivered to a needed location inside a blood vessel because such a stent cannot navigate through a tortuous and small vessel lumen.

WO 99/40876 discloses a vascular stent, having parallel expansion columns. Each expansion column is made of expansion struts forming a plurality of expansion strut pair loops that coupled adjacent individual expansion struts. Two adjacent expansion strut pair loops share a common expansion strut. Each expansion strut is made of two or three expansion strut segments. The expansion strut segments of one expansion strut are arranged such that their longitudinal axes intersect each other.

The problem of the invention is to provide a vascular stent that is very flexible for delivery, with a good vessel coverage when deployed and that provides a uniform force distribution to the vessel wall.

The problem is solved according to claim 1. Preferred embodiments are subject matter of the dependent claims.

### SUMMARY OF THE INVENTION

Various embodiments of a stent include a combination of maximum possible flexibility and conformability in the stent, full vessel coverage with optimal metal fraction, evenly expanding stent struts, excellent radial strength and radiopacity, and smooth surface modulations in both delivery and deployed phases of the stent life cycle. To arrive at these goals, many detailed new innovations are added to the expansion and connecting strut designs of the stent. Expansion strut design is largely responsible for radial strength and radiopacity, while connecting strut design is largely responsible for flexibility and smooth surface modulations. Full vessel coverage and uniform stent expansion are largely from interaction between expansion and connecting struts. Various embodiments of the stent demonstrate a balance among these multiple qualities, using smart expansion struts and flexible connecting struts in a seamlessly integrated stent network.

Various embodiments of the stent are specifically designed to be both very flexible and fully cover vessel surface inside the vascular lumpen. The stent can have both characteristics of vessel coverage and flexibility, particularly for coronary use.

Various embodiments of a stent are well designed for both the delivery phase and the deployed phase of the stent life cycle. Both flexibility and good vessel coverage are in a right balance in various embodiments of the stent have. Various embodiments of the stent include certain configurations in expansion and connecting struts of the stent.

One embodiment includes a first expansion column, a second expansion column, and a first connecting strut column. The first expansion column and the second expansion column include individual expansion struts. The individual expansion struts form a plurality of expansion strut pairs. Two adjacent expansion strut pairs share a common strut. The first connecting strut column includes a plurality of individual connecting struts. The plurality of individual connecting struts can couple the first and second expansion columns. Each individual connecting strut can include at least six pivot points. The invention includes a first expansion column, a second expansion column, and a first connecting strut column. The first expansion column and the second expansion column include individual stair-step expansion struts. The individual stair-step expansion struts form a plurality of expansion strut pair loops. Two adjacent expansion strut pair loops share a common stair-step expansion strut. The first connecting strut column can include a plurality of individual symmetrical geometry connecting struts. Each of an end of an individual connecting strut can extend ipsilaterally from sides of expansion strut pair loops of the first and second expansion columns.

### BRIEF DESCRIPTION OF DRAWINGS

Figure 1 shows a side elevation view of an embodiment of a stent, such as a tubular stent.
Figure 2 shows an isometric view of an embodiment of a sent, such as a tubular stent.
Figure 3 shows a cut-open view of an embodiment of a stent. Various expansion columns and connecting strut columns are shown.
Figure 4A shows a magnified view of a middle section of an embodiment of a stent, such as a stent of Figures 1, 2, and/or 3. Some details are shown of expansion columns.
Figure 4B shows a magnified view of a middle section of an embodiment of a stent, such as a stent of Figures 1, 2, and/or 3. Some details are shown of connecting strut columns conjoined with expansion columns.
Figure 5A shows a magnified view of an end section of one embodiment of a stent, such as a stent of Figures 1, 2, and/or 3. Some details are shown of an end expansion column.
Figure 5B shows a magnified view of an end section of one embodiment of a stent, such as a stent of Figures 1, 2, and/or 3. of present invention in a two-dimensional view. Some details are shown of different connecting strut columns conjoined with expansion columns.

### DETAILED DESCRIPTION

Some embodiments of stents can be in a state, such as one or more of a non-expanded state, an expanded state, a crimped state, and a non-crimped state.

Some embodiments of stents can include one or more of a first expansion column, a second expansion column, a third expansion column, a first connecting strut column, and a second connecting strut column.

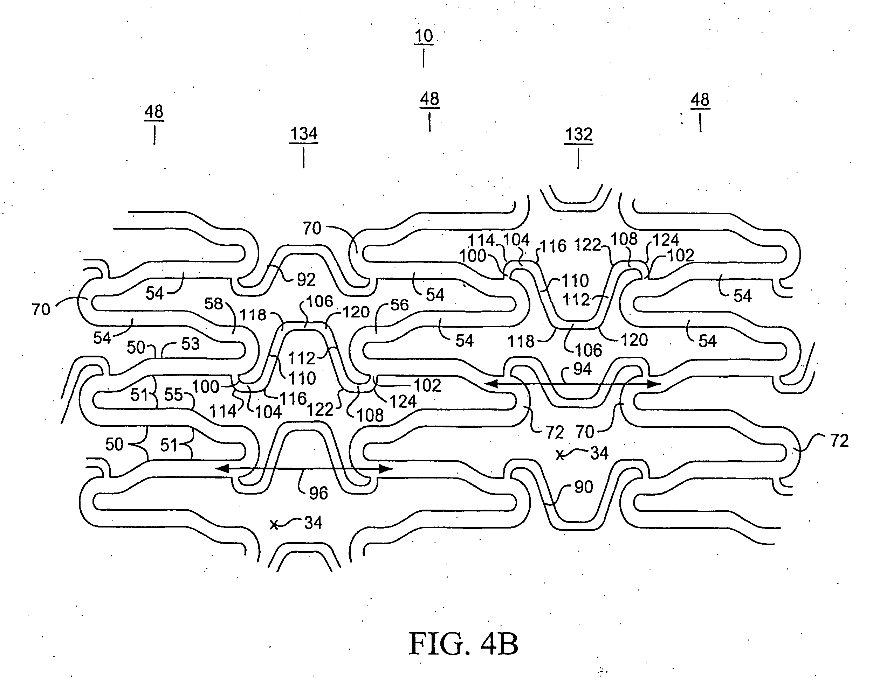

The first expansion column, the second expansion column, and/or the third expansion column can include individual expansion struts forming a plurality of expansion strut pairs. Figure 4B shows examples of individual expansion struts 50 and expansion strut pairs 51. In some embodiments of the stent, one expansion strut of an expansion strut pair can have a stair-step segment at a proximal end and the other expansion strut of the expansion strut pair can have a stair-step segment at a distal end. Figure 4B shows examples of one expansion strut 53 of an expansion strut pair 51 having a stair-step segment at a proximal end and the other expansion strut 55 of the expansion strut pair 51 having a stair-step segment at a distal end. In some embodiments of the stent, two adjacent expansion strut pairs share a common strut.

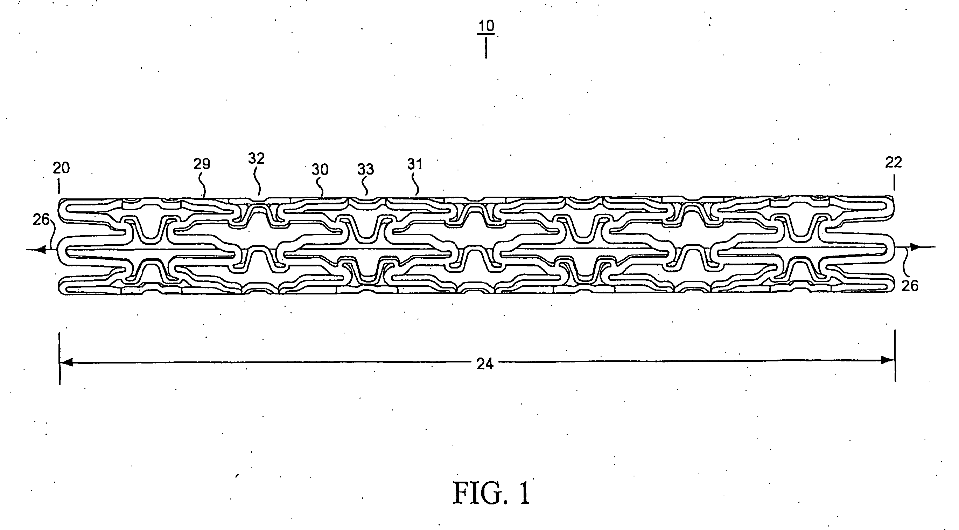

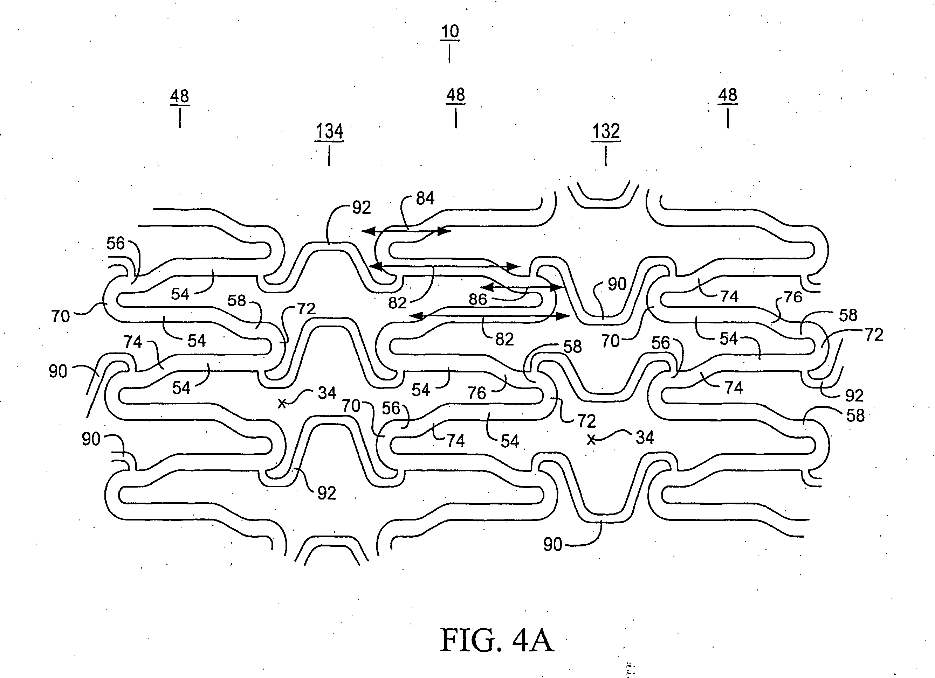

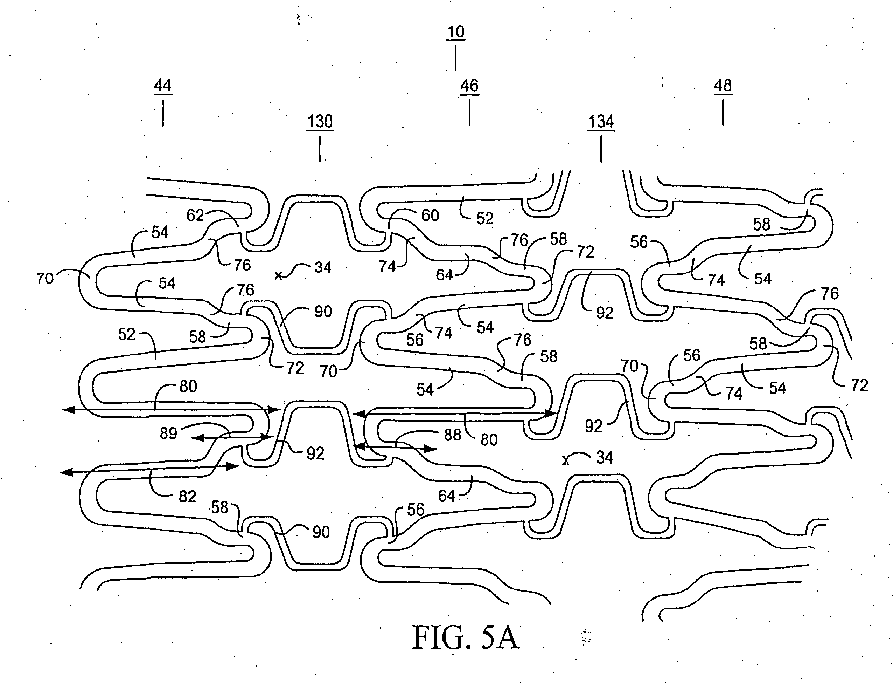

The first connecting column and/or the second connecting column can include a plurality of individual connecting struts. The plurality of individual connecting struts for at least the first connecting column can couple the first and second expansion columns. In various embodiments of the stent, each connecting strut includes one or more of: at least some number of pivot points, at least some number of sections, bilateral short stems, a geometrical configuration, at least some number of radii of curvature, a center section, and a longitudinal axis. For example, each connecting strut can include at least six pivot points; at least four, five, six, or seven sections; and/or at least three, four, five, or six radii of curvature. Figure 4B shows examples 114, 116, 118, 120, 122, and 124 of pivot points each having some radius of curvature. Bilateral short stems can be ipsilaterally conjoined to an expansion strut of the first expansion column and to an expansion strut of the second expansion column. Figure 4B shows examples of bilateral stems 100 and 102 on proximal and distal ends respectively of a connecting strut. Exemplary geometrical configurations include a symmetrical one and/or a quasi M-frame one. Figure 4A shows examples of connecting struts 90 and 92 which have both a symmetrical geometrical configuration and a quasi M-frame geometrical configuration. The longitudinal axis can be non-perpendicular to a longitudinal axis of the stent, substantially perpendicular to a longitudinal axis of the stent, and/or substantially parallel to a longitudinal axis of the stent. Figures 1, 2, and 3 show examples of a longitudinal axis 26 of a stent 10. Examples of longitudinal axes of connecting struts are shown in Figure 4B as axes 94 and 96. Other examples of axes are in Figure 4A shown as axes 82, 84, and 86 and in Figure 5A as axes 80, 82, 88, and 89.

In some embodiments of the stent, each connecting strut can be invaginated and/or inverted into a connector space between expansion strut pairs between the first and second expansion columns. Figure 4A shows examples of connecting struts 90 and 92 which are invaginated and inverted into the connector space between expansion strut pairs.

In some embodiments of the stent, each connecting strut can be ipsilaterally coupled to an expansion strut of the first expansion column and to an expansion strut of the second expansion column. Figure 4A shows examples of connecting struts 90 and 92 which are ipsilaterally coupled between expansion struts of different expansion columns.

In various embodiments of the stent, at least one connecting strut has a geometric configuration, such as an asymmetrical geometric configuration and/or a quasi M-frame geometric configuration.

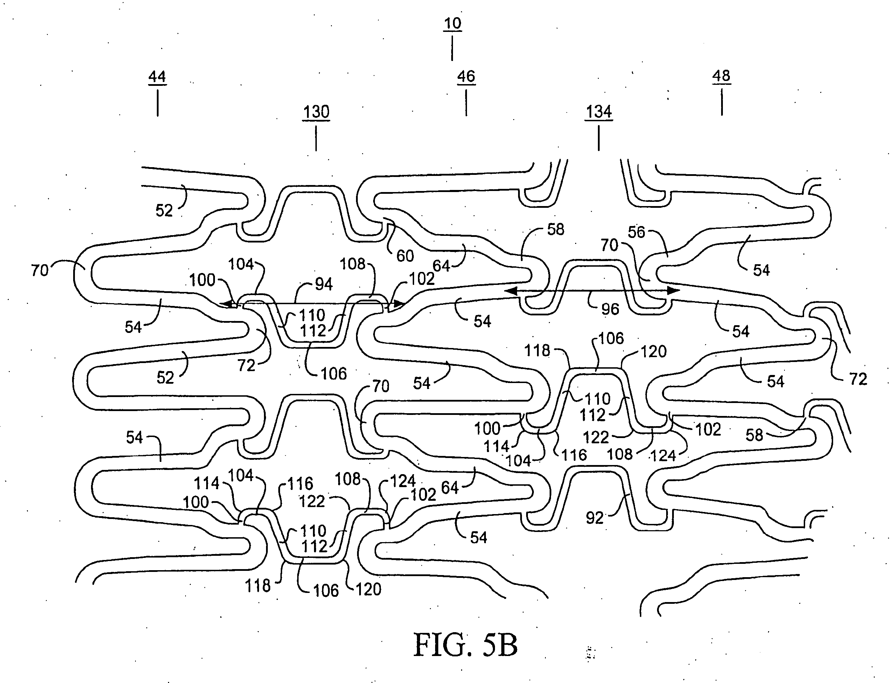

In some embodiments of the stent the center section can have a substantially truncated conical configuration. In some embodiments of the stent all center sections of the connecting struts extend in a first direction. In some embodiments of the stent the center section of at least a portion of the connecting struts extend in a first direction and/or extend in a second direction. Figure 5A and 5B show examples of a connecting strut column 130 having center sections that extend in a first direction, and a connecting strut column 134 having center sections that extend in a first direction and a second direction.

Some embodiments of the stent include a plurality of expansion columns. The plurality of expansion columns can be coupled by a plurality of connecting strut columns. Each connecting strut can have a longitudinal axis. In some embodiments of the stent, substantially every longitudinal axis of a connecting strut in a connecting column is parallel to the longitudinal axis of the connecting struts in that column. Each connecting strut can have, for example, at least six pivot points.

Some embodiments of the stent include a first end expansion column and a second end expansion column. The first end expansion column and the second end expansion column can define a proximal and a distal end of the stent. The first end expansion column and the second end expansion column can be mirror images of each other.

Some embodiments of the stent include a plurality of cells. Cells can have asymmetrical geometries ad/or symmetrical geometries. Some geometric shapes have a semi- hexagonal geometry in a nominally expanded state, such as within operating parameters. Cells can be defined by the first expansion column, the second expansion column, and the first connecting strut column. Cells can be defined by the second expansion column, the third expansion column and the second connecting strut column. Cells can have evenly spaced geometric shapes.

Some embodiments of stents can include one or more of a first expansion column, a second expansion column, and a first connecting strut column.

The first expansion column and/or the second expansion column can include individual stair-step expansion struts forming a plurality of expansion strut pair loops. In some embodiments of the stent, expansion strut pair loops couple adjacent individual expansion struts. In some embodiments of the stent, two adjacent expansion strut pair loops share a common stair-step expansion strut. ,

The first connecting strut column can include a plurality of individual symmetrical geometry connecting struts. The plurality of individual symmetrical geometry connecting struts for at least the first connecting column can couple the first and second expansion columns. In various embodiments of the stent, each symmetrical geometry connecting strut includes one or more of: at least some number of pivot points, at least some number of sections, bilateral short stems, a geometrical configuration, at least some number of radii of curvature, a center section, and a longitudinal axis. For example, each symmetrical geometry connecting strut can include at least six pivot points; at least four, five, six, or seven sections; and/or at least three, four, five, or six radii of curvature. Bilateral short stems can be ipsilaterally conjoined to an expansion strut of the first expansion column and to an expansion strut of the second expansion column. Exemplary geometrical configurations include a symmetrical one and/or a quasi M-frame one. The longitudinal axis can be non-perpendicular to a longitudinal axis of the stent, substantially perpendicular to a longitudinal axis of the stent, and/or substantially parallel to a longitudinal axis of the stent.

In some embodiments of the stent, each symmetrical geometry connecting strut can be invaginated and/or inserted into a connector space between expansion strut pair loops between the first and second expansion columns.

In some embodiments of the stent, each symmetrical geometry connecting strut can be ipsilaterally coupled to an expansion strut of the first expansion column and to an expansion strut of the second expansion column.

In various embodiments of the stent, at least one symmetrical geometry connecting strut has a geometric configuration, such as an asymmetrical geometric configuration and/or a quasi M-frame geometric configuration.

In some embodiments of the stent the center section can have a substantially truncated conical configuration. In some embodiments of the stent all center sections of the symmetrical geometry connecting struts extend in a first direction. In some embodiments of the stent the center section of at least a portion of the symmetrical geometry connecting struts extend in a first direction and/or extend in a second direction.

Some embodiments of the stent include a plurality of expansion columns. The plurality of expansion columns can be coupled by a plurality of connecting strut columns. Each symmetrical geometry connecting strut can have a longitudinal axis. In some embodiments of the stent, substantially every longitudinal axis of a symmetrical geometry connecting strut in a connecting column is parallel to the longitudinal axis of the symmetrical geometry connecting struts in that column. Each symmetrical geometry connecting strut can have, for example, at least six pivot points.

Some embodiments of the stent include a first end expansion column and a second end expansion column. The first end expansion column and the second end expansion column can define a proximal and a distal end of the stent. The first end expansion column and the second end expansion column can be mirror images of each other.

In some embodiments of the stent, individual expansion struts of the first and second expansion column can form a plurality of expansion strut pair loops that couple adjacent individual expansion strut pair loops. In some embodiments of the stent, adjacent individual expansion strut pair loops can be coupled in a symmetrical geometry.

In some embodiments of the stent, expansion strut pair loops of the first and second expansion columns can be aligned. For example, the alignment can be in a peak-to-valley geometry, in a valley-to-peak geometry, and/or in a peak-to-peak geometry.

Expansion struts can include zigzag cycles in a ring shape to form an expansion ring otherwise known as an expansion column. These expansion columns are largely responsible for optimal crimping, even and smooth expanding, and radial strength. Expansion columns by themselves may not be flexible. Each zigzag cycle in an expansion column can have a pair of expansion struts. Two expansion struts can be conjoined, for example, by a joining loop section at a proximal or a distal end. Such pairing, including conjoining at an alternating proximal to distal and distal to proximal sequence, continues in one embodiment twelve (12) times seamlessly around the circumference of an expansion column, in the one embodiment that has six zigzag cycles around the circumference of the expansion column.

Various embodiments of the stent can include one or more of several different types of expansion columns. A first end expansion column in a proximal end can be a mirror image of a second expansion column in a distal end. A second expansion column nearest to the proximal end expansion column can have its mirror image in an expansion column next to a distal end expansion column. Such expansion columns can transition to a middle expansion column. The middle of the stent can include another type of expansion column that can repeat to make up the rest of the middle section of the stent of a predetermined length.

In some embodiments of the stent, the proximal end expansion column can include one or more types of expansion strut patterns forming different kinds of expansion strut pairs around the circumference of the proximal end expansion column. Such types include an expansion strut with a distal stepped-down section, an expansion strut with a straight-line configuration, and an expansion strut with a distal stepped-up section. A distal end expansion column can be a mirror image of the proximal end expansion column. Different types of expansion struts can be arranged in certain sequence. Joining loop sections can form expansion strut pair loops at a proximal end and at a distal end, for example in an alternating sequence.

An expansion column nearest to a proximal end expansion column and an expansion column nearest to a distal end expansion column can be mirror images. One or more types of expansion strut patterns are possible. Various embodiments of the stent can include one or more of: an expansion strut with a stepped-down section at a proximal end, an expansion strut with a stepped-down section at a distal end, an expansion strut with a straight line configuration, and an expansion strut with a stepped-up section at a proximal end and a stepped-down section at a distal end. Such types of expansion struts can be arranged in certain sequence. Joining loop sections can form expansion strut pair loops at a proximal end and at a distal end, for example in an alternating sequence. An expansion column nearest to a proximal end expansion column and an expansion column nearest to a distal end expansion column can have the same conjoining of expansion pair loops.

Expansion columns in the middle may have one or more types of expansion struts, such as an expansion strut with a stepped-down section at a proximal end, and an expansion strut with a stepped-down section at a distal end. A pair of these types can be conjoined by, for example, a joining loop section at a proximal end or at a distal end, making expansion strut pair loops in a proximal end or in a distal end in an alternating sequence.

Various embodiments of the stent can include multiple types of expansion columns. Particular configurations of an expansion strut pair and expansion columns can be created for specific performance purposes. The short stepped-up or stepped-down part and the longer straight part in an expansion strut with a sloped transitional zone between a long and short part can provide distinct expansion characteristic, smooth surface modulation effects, and well-formed crimping space to stent performance. A short stepped-down or stepped-up section of an expansion strut can be where a connecting strut can conjoin on a side of an expansion strut pair loop. A connecting strut can conjoin with an expansion strut as a direct extension from a side of an expansion strut pair loop and can be an integral stent structure, rather than a separate structure added, welded or attached. Separate terminology for stent elements, for example, expansion and connecting struts, conveniently describes the anatomy and function of various stent portions, and may not imply that previously separate elements are subsequently connected together.

Connecting struts can have a geometric configuration, for example a symmetrical quasi M-frame configuration. In the symmetrical quasi M-frame configuration, the center element can have an invaginated truncated conical shape (or a trapezoid shape), and/or with outer arms truncated short. A longitudinal axis of a connecting strut can align with a longitudinal axis of a stent. Various embodiments of the stent can have connecting struts with one or more of horizontal segments, slant-vertical sections, and short outer arm-end sections or bilateral short stems, with some number of pivot points. In one embodiment, connecting struts include three horizontal segments, two long slant-vertical sections, and two bilateral short stems, with six pivot points. The pivot points of a connecting strut have some radii of curvature of a varying degree to make the corners smooth with a good surface modulation. In one embodiment, the pivot points makes the stent flexible while inducing a very smooth surface geometry.

Connecting struts can conjoin on ipsilateral sides of expansion strut pair loops on each end. The center element, such as of an upside down trapezoid (or truncated conical shape) of the connecting strut can be invaginated into the connector space between the two apposing expansion strut pair loops that are, for example, aligned in a mirror image pattern. Some embodiments of the stent do not protrude into the main cell space. One configuration, a quasi M-frame connecting strut, divides the connector space into multiple portions. The bilateral short stems can be conjoined on ipsilateral sides of the apposed expansion strut pair loops, while the center element, the truncated conical shape or the trapezoid shape, can be invaginated into the connector space between the two apposed expansion strut pair loops.

In some embodiments of the stent, when two bilateral short stems of a connecting strut conjoins expansion strut pair loops on ipsilateral sides, the connecting strut can conjoin to the two apposing expansion strut pair loops on each side of the connecting strut. A stepped-down or a stepped-up section of an expansion strut can give a connecting strut a well-planned space for crimping. Conjoining of a connecting strut on ipsilateral sides, along with an invaginated center section or sections into a connector space with multiple pivot points can create flexibility, smooth surface modulation, conformability, cell geometry (for example, hexagonally expanded) and a well formed full vessel coverage stent net mesh without an excessive metal fraction.

In some embodiments of the stent, an end of a connecting strut can be conjoined to an expansion strut pair loop, making a ratio of expansion struts to connecting struts two to one.

In some embodiments of the stent, when the expansion columns and connecting columns are conjoined as a single unit, the stent can have a continuous, unbroken cylindrical form without breaks or delinking around the circumference and along the length of the stent. The unbroken link between the expansion and connecting struts can make regular and evenly spaced asymmetrical cells. The cell size can be maximized or minimized by programming of the stent (design) platform, as the clinical or application requirements may dictate.

Figure 1 shows one embodiment of a stent 10 in side elevation view, with a first expansion column 29, a second expansion column 30, a third expansion column 31, a first connecting strut column 32, and a second connecting strut column 33. The stent 10 has a proximal end 20 and a distal end 22. The stent 10 can have a tubular or cylindrical structure. The stent 10 can have a longitudinal length 24 and a longitudinal axis 26.

In some embodiments of the stent, an expansion column can be a zigzag and/or corrugated ring configuration of expansion struts. An expansion column, for example expansion column 30, in a stent 10 can be an unbroken circular ring. Multiple expansion strut columns can be interconnected with connecting struts continuously along the longitudinal axis 26 of the stent 10 in an unbroken manner to form a stent 10 having a tubular shape. The interconnections among expansion columns and connecting strut columns enclose spaces, or cells, formed by expansion struts and connecting struts. In the embodiment shown in Figure 1, many cells have symmetrical geometry, for example the middle of the stent 10, but some cells, for example near proximal end 20 and distal end 22, can have asymmetrical geometry.

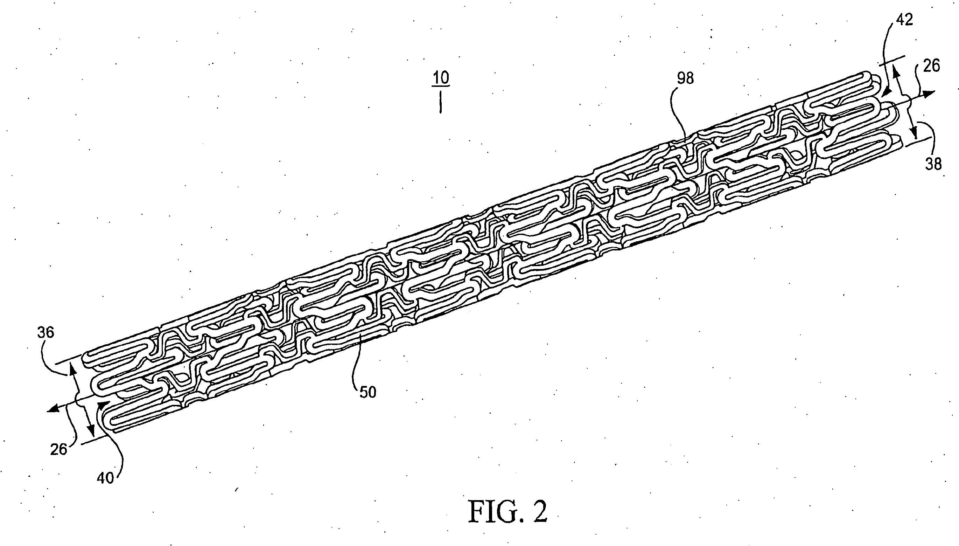

Figure 2 shows one embodiment of a stent 10 in isometric view. A back half of the stent 10 can be seen through the front half of the stent 10. The shown embodiment of the stent 10 has a tubular structure with a central lumen, a proximal opening 40, and a distal opening 42. Stent cells 34 include open spaces in the network of expansion struts and connecting struts. The lumen includes the central, open tunnel formed by the stent. The stent 10 has two different diameters, including an outer diameter 36 and an inner diameter 38, having a difference of a thickness of the stent 10. Both the outer diameter 36 and inner diameter 38 can change as the stent 10 goes through a crimping stage, when the diameters 36 and 38 are narrowed, and through a deployed stage, when the diameters 36 and 38 are expanded.

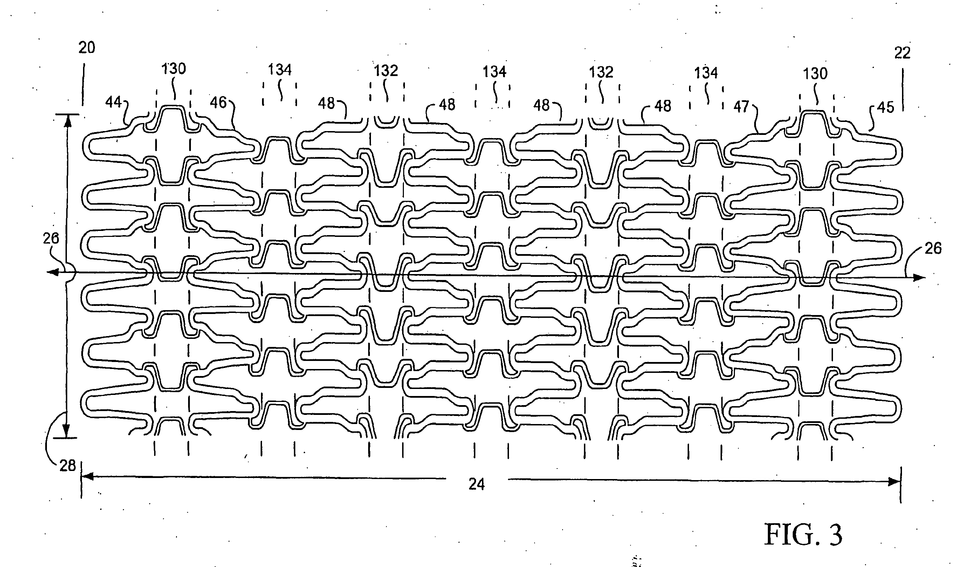

Figure 3 shows one embodiment of a stent 10 in cut-open view. The stent 10 has a proximal end 20 and a distal end 22. This view of the stent 10 is a scale drawing for a 15 mm coronary stent. There are eight expansion columns and seven connecting strut columns. At the proximal end 20 are two different expansion columns 44 and 46, which are mirror images of two expansion columns 45 and 47 at the distal end 22. In the middle of the stent 10, there are four identical expansion columns 48. Interconnecting with eight expansion columns along the longitudinal axis 26 of the stent 10 are seven connecting strut columns. The first connecting strut column 130 in a proximal end and the last connecting strut column 130 are mirror images. In the middle of the stent 10 are two upright connecting strut columns 132 and three upside down connecting strut columns 134. There are a total of 49 cells of six different geometric configurations. Some cells have symmetrical geometry and some have asymmetrical geometry.

Expansion columns 44, 46, 48, 47 and 45 are vertically arranged with expansion strut pair loops aligned peak-to-peak. A short distal step-down segment of one expansion column is matched with a short proximal step-down segment of another expansion column. In the middle of the stent 10, a peak-to-peak matching alignment pattern of strut pair loops repeats. Geometric configurations of expansion columns 44 and 46 in the proximal end 20 and expansion columns 47 and 45 in the distal end 22 are mirror images from expansion columns 48 in the middle of the stent. Peak-to-peak alignment of expansion strut pair loops of distal and proximal step-down segments are consistent throughout the stent 10.

Connecting strut columns 130, 132 and 134 interconnect expansion columns 44, 46, 48, 47 and 45 in a continuous and unbroken manner along the length 24 and around the circumference 28 of the stent 10. The first and last connecting strut columns 130 use both upside down and upright quasi M-frame connecting struts. In the middle of the stent 10, connecting strut columns 132 use upright quasi M-frame connecting struts, whereas connecting strut columns 134 have upside down quasi M-frame connecting struts. The quasi M-frame connecting struts are mounted on the ipsilateral sides of two apposed expansion strut pair loops with a distal and proximal step-down segments. This apposed arrangement of distal versus proximal step-down segments of the corrugated loops of expansion columns 30 is for a smooth and efficient crimping space for proximal and distal bilateral short stems of quasi M-frame connecting struts in the stent 10.

The stent 10 in Figure 3 has the proximal end 20 on the left and the distal end 22 on the right. The stent 10 has a length 24 horizontally and a circumference 28 vertically, with a longitudinal axis 26 horizontally along the length 24 from the proximal end 20 to the distal end 22.

A width (horizontal dimension) of expansion columns is wider than a width of connecting strut columns. However, a width of a connecting strut column could be made the same or larger than a width of an expansion column. The variation of width ratio between a connecting strut column and an expansion column are within the scope of present invention of stent 10. The number of expansion strut cycles in an expansion column and the number of connecting struts in a connecting strut column can be made variably different. Variable numbers of making expansion strut cycles and connecting struts are within the scope of the present invention of the stent 10.

Figure 4A shows a magnified view of a middle section of one embodiment of a stent 10. Figure-4A shows identical expansion columns 48. Each expansion column 48 can have six cycles of continuous, unbroken expansion strut pair loops witch six loops on a proximal end and six loops on a distal end. Each expansion strut pair loop in an expansion column 48 can include a stair step expansion strut 54 with a stepped-down short segment 56 in a proximal end and a stair step expansion strut 54 with a short stepped-down segment 58 in a distal end, in a regularly alternating sequence. The embodiment of stent 10 of Figure 3 includes twelve stair step expansion struts 54 in an expansion column 48. A pair of stair step expansion struts 54 is conjoined by a joining loop 70 in a proximal end and a pair of stair step expansion struts 54 is conjoined by a joining loop 72 in a distal end. When a pair of stair step expansion struts 54 is conjoined by a joining loop 70 or 72, a loop is formed.

An expansion strut 54 can have a longer straight segment and a shorter stepped down segment 56 in a proximal end. A transitional slope 74 can be between a stepped down proximal segment 56 and a straight segment in a stair step expansion strut 54. Likewise, a transitional slope 76 can be between a stepped down distal segment 58 and a straight segment in a stair step expansion strut 54. Expansion strut pair loops of an expansion column 48 can be identical in expansion columns marked 48.

In an expansion column 48, a straight segment of expansion strut 54 can have a longitudinal axis 82 in a horizontal direction. Similarly, a proximal short stepped down segment 56 can have a longitudinal axis 84, which also lies horizontally and roughly parallel with an axis 80 although the axis 84 does not have to be parallel with the axis 82. A distal short stepped down segment 58 has a longitudinal axis 86, which also lies horizontally and may be parallel with the axis 80, although the axis 86 does not have to be parallel with an axis 82.

Expansion columns 48 can be vertically aligned, with proximal peaks 70 of expansion strut pair loops of one expansion column 48 apposed with distal peaks 72 of expansion strut pair loops of adjacent expansion column 48. Short stepped down segments 56 and 58 of adjacent expansion columns 48 are aligned on the ipsilateral, or same sides. Similarly, long straight segments of expansion struts 54 in an adjacent expansion column 48 can also be aligned on the ipsilateral sides. The ipsilateral apposition of stepped down segments 56 and 58 between two adjacent expansion columns 48 allows for symmetrical conjoining of a quasi M-frame connecting strut to adjacent expansion columns 48.

As expansion columns are arranged in Figure-4A, a longitudinal axis 82 of a stair step expansion strut 54 in an expansion column 48 is roughly parallel with a longitudinal axis 82 of a stair step expansion strut 54 in adjacent expansion column 48, although non-paralleling of these two axis 82 in expansion struts 54 in adjacent expansion columns does not have to be so.
This variation is within the scope of present invention of stent 10.

An upright quasi M-frame connecting strut 90 can be conjoined on the ipsilateral sides of expansion strut pair loops in peak-to-peak apposition on the stepped down segments of the expansion strut pair loop of adjacent expansion columns 48. The center element of quasi M-frame connecting strut 90 can be located within the confines of the connector space between two apposed expansion strut pair loops of adjacent expansion columns 48. An upside down quasi M-frame connecting strut 92 can be conjoined on the ipsilateral sides of expansion strut pair loops in peak-to-peak apposition on the straight longer segment side of expansion strut pair loops of adjacent expansion columns 48. The center element of upside down quasi M-frame connecting strut 92 can also be located within the confines of the connector space between two apposed expansion strut 54 pair loops of adjacent expansion columns 48.

Figure 4B shows a magnified view of a middle section of one embodiment of a stent 10. An upright quasi M-frame connecting strut 90 conjoins two adjacent expansion columns 48. A connecting strut 90 has a longitudinal axis 94, which lies horizontally along the same direction as longitudinal axis 26 of the stent 10.

An upright quasi M-frame connecting strut 90 has a proximal bilateral short stem 100 in the proximal end and a distal bilateral short stem 102 in the distal end. These two stems are anchoring roots a connecting strut 90 to conjoin, on ipsilateral sides, stepped down short segments 56 and 58 of apposed expansion strut pair loops of two adjacent expansion columns 48. A quasi M-frame connecting strut 90 has a symmetrical geometric shape. There are three horizontal segments 104, 106 and 108. A horizontal segment 104 is an extension from a proximal stem 100 through a radius of curvature 114. A distal horizontal segment 108 along with a distal stem 102 and a radius of curvature 124 is a mirror image of a proximal horizontal segment. A middle horizontal segment 106 is at the base of a truncated cone of the quasi M-frame connecting strut 90. On both sides of a middle horizontal segment 106 are two mirror image vertical slant segments 110 and 112. A proximal vertical slant segment 110 is an extension of proximal horizontal segment 104 through a radius of curvature 116 and is an extension of middle horizontal segment 106 through a radius of curvature 118. Likewise, a distal vertical slant segment 112 is an extension of a middle horizontal segment 106 through a radius of curvature 120 and is an extension of distal horizontal segment 108 through a radius of curvature 112. There are six radii of curvature 114, 116, 118, 120, 122, and 124 in a quasi M-frame connecting strut 90. These six radii of curvatures serve as flexibility pivot points in the connecting strut 90, so that the stent 10 can have more flexibility. The structure of a quasi M-frame connecting strut 90 can be substantially or entirely confined inside an imaginary "connector space" between two apposed expansion strut pair loop peaks 70 and 72 of adjacent expansion columns 48. The central element, such as a truncated cone, of a quasi M-frame connecting strut 90, can be inverted or invaginated into a connector space between apposed expansion strut pair loops of adjacent expansion columns 48, instead of projecting substantially into the free space of the stent cell 34. This can enhance stent crimping as well as smooth surface modulation during a delivery phase of stent implant procedure.

The upside down quasi M-frame connecting strut 92 is a reverse image of an upright quasi M-frame connecting strut 90. A quasi M-frame connecting strut 92 in a connector space 134 is similar to an upright quasi M-frame connecting strut 90, but having an upside down orientation, rather than an upright orientation of the upright quasi M-frame connecting strut 90. Designations of an upside down quasi M-frame connecting strut are similar to that of an upright quasi M-frame connecting strut 92. In the middle of the stent 10, connecting strut columns 32 can alternate between upright quasi M-frame connecting strut column 132 and upside down quasi M-frame connecting strut column 134, while expansion columns 48 can repeat a same configuration. Upside down connecting struts 92 in connecting strut columns 134 can be conjoined on the ipsilateral sides of long straight segments of expansion struts 54 of expansion strut pair loop peaks 70 and 72 of adjacent expansion columns 48.

The total length of an M-frame 90 can be substantially longer than the width of a connecting strut column space 132. This can compensate a foreshortening of the stent 10 when expanded, and enhance the flexibility of connecting strut column 132.

In connecting strut column 132, a quasi M-frame connecting strut 90 is conjoined to ipsilateral sides on the proximal or distal stepped down segments 56 an 58 of two apposed expansion strut pair loops of adjacent expansion columns 48.

Figure 5A shows a magnified view of an end section of one embodiment of a stent 10, such as a proximal end 20 of stent 10. This Figure focuses on the details of the expansion columns 44, 46, and 48.

Figure 5B shows a magnified view of an end section of one embodiment of a stent 10, with details of connecting strut columns 130 and 134.

## Claims

1. A stent in a non-expanded state, comprising:
• a first expansion column (48) including individual stair-step expansion struts (54) forming a plurality of expansion strut pair loops that couple adjacent individual expansion struts (54), wherein two adjacent expansion strut pair loops share a common stair-step expansion strut (54);
• a second expansion column (48) including individual stair-step expansion struts (54) forming a plurality of expansion strut pair loops that couple adjacent individual expansion struts (54), wherein two adjacent expansion strut pair loops share a common stair-step expansion strut (54);
• and a first connecting strut column (32, 134) including a plurality of individual symmetrical geometry connecting struts (92), wherein each of an end of an individual connecting strut (92) extends ipsilaterally from sides of expansion strut pair loops of the first and second expansion columns (48),
• **characterized in that** each expansion pair loop includes a first stair step expansion strut (54) and a second stair step expansion strut (54).

2. The stent of claim 1, wherein each connecting strut (92) of the first connecting strut column (32, 134) includes bilateral short stems (100, 102) that are ipsilaterally conjoined to an expansion strut (54) of the first expansion column (48) and to an expansion strut (54) of the second expansion column (48).

3. The stent of claim 1, wherein at least one connecting strut (92) of the first connecting strut column (32, 134) has an asymmetrical geometric configuration.

4. The stent of claim 1, wherein each connecting strut (92) of the first connecting strut column (32, 134) has at least six radii of curvature.

5. The stent of claim 1, wherein expansion strut pair loops of the first and second expansion columns (48) are aligned in a valley-to-peak geometry.

6. The stent of claim 1, wherein expansion strut pair loops of the first and second expansion columns (48) are aligned in a peak-to-peak geometry.

7. The stent of claim 1, wherein the center section of at least a portion of the connecting struts (92) in the first connecting strut column (32, 134) extend in a first direction and the center section of at least a portion of the connecting struts (92) in the first connecting strut column (32, 134) extend in a second direction.

8. The stent of claim 1 **characterized in that** the stair step expansion struts (54) include a first section, a second section and a third section disposed between the first and second sections, the first and second sections being parallel to one another, wherein the third section is not parallel to the first section and to the second section.

9. The stent of claim 1 wherein some of the stair-step expansion struts (54) have a stepped-up section at a proximal end.

10. The stent of claim 1 wherein some of the stair-step expansion struts (54) have a stepped-up section at a distal end.

11. The stent of claim 1 comprising expansion struts (54) with a stepped-up section at a proximal end and a stepped-down section (58) at a distal end.

12. The stent of claim 1 comprising straight line expansion struts.

13. The stent of any of the preceding claims, wherein each stair-step expansion strut (54) comprises a proximal end, a distal end, and a stair-step segment (56, 58) positioned adjacent either the proximal end or the distal end.

14. The stent of any of the preceding claim, wherein each strut pair loop includes a stair-step expansion strut with a stair-step at a distal end and a stair-step expansion strut with a stair-step at a proximal end.

## Patentansprüche

1. Stent in einem nicht expandierten Zustand, umfassend:
• eine erste Expansionssäule (48), umfassend einzelne treppenförmige Expansionsstreben (54), die eine Mehrzahl Expansionsstrebenpaarschleifen bilden, die benachbarte einzelne Expansionsstreben (54) koppeln, wobei zwei benachbarte Expansionsstrebenpaarschleifen eine gemeinsame treppenförmige Expansionsstrebe (54) teilen;
• eine zweite Expansionssäule (48), umfassend einzelne treppenförmige Expansionsstreben (54), die eine Mehrzahl Expansionsstrebenpaarschleifen bilden, die benachbarte einzelne Expansionsstreben (54) koppeln, wobei zwei benachbarte Expansionsstrebenpaarschleifen eine gemeinsame treppenförmige Expansionsstrebe (54) teilen;
• und eine erste Verbindungsstrebensäule (32, 134), umfassend eine Mehrzahl einzelner Verbindungsstreben (92) von symmetrischer Geometrie , wobei sich jedes Ende einer einzelnen Verbindungsstrebe (92) gleichseitig von Seiten von Expansionsstrebenpaarschleifen der ersten und zweiten Expansionssäulen (48) erstreckt,
• **dadurch gekennzeichnet, dass** jede Expansionspaarschleife eine erste treppenförmige Expansionsstrebe (54) und eine zweite treppenförmige Expansionsstrebe (54) umfasst.

2. Stent nach Anspruch 1, wobei jede Verbindungsstrebe (92) der ersten Verbindungsstrebensäule (32, 134) bilaterale kurze Stege (100, 102) umfasst, die gleichseitig an einer Expansionsstrebe (54) der ersten Expansionssäule (48) und an einer Expansionsstrebe (54) der zweiten Expansionssäule (48) befestigt sind.

3. Stent nach Anspruch 1, wobei mindestens eine Verbindungsstrebe (92) der ersten Verbindungsstrebensäule (32, 134) eine asymmetrische geometrische Ausgestaltung aufweist.

4. Stent nach Anspruch 1, wobei jede Verbindungsstrebe (92) der ersten Verbindungsstrebensäule (32, 134) mindestens sechs Krümmungsradien aufweist.

5. Stent nach Anspruch 1, wobei Expansionsstrebenpaarschleifen der ersten und zweiten Expansionssäulen (48) in einer Spitze-Tal-Geometrie ausgerichtet sind.

6. Stent nach Anspruch 1, wobei Expansionsstrebenpaarschleifen der ersten und zweiten Expansionssäulen (48) in einer Spitze-Spitze-Geometrie ausgerichtet sind.

7. Stent nach Anspruch 1, wobei sich der Mittelabschnitt mindestens eines Teils der Verbindungsstreben (92) in der ersten Verbindungsstrebensäule (32, 134) in einer ersten Richtung erstreckt und sich der Mittelabschnitt mindestens eines Teils der Verbindungsstreben (92) in der ersten Verbindungsstrebensäule (32, 134) in einer zweiten Richtung erstreckt.

8. Stent nach Anspruch 1, **dadurch gekennzeichnet, dass** die treppenförmigen Expansionsstreben (54) einen ersten Abschnitt, einen zweiten Abschnitt und einen dritten Abschnitt, der zwischen dem ersten und zweiten Abschnitt angeordnet ist, umfassen, wobei der erste und zweite Abschnitt parallel zueinander sind, wobei der dritte Abschnitt nicht parallel zum ersten Abschnitt und zum zweiten Abschnitt ist.

9. Stent nach Anspruch 1, wobei einige der treppenförmigen Expansionsstreben (54) an einem proximalen Ende einen erhöhten Abschnitt aufweisen.

10. Stent nach Anspruch 1, wobei einige der treppenförmigen Expansionsstreben (54) an einem distalen Ende einen erhöhten Abschnitt aufweisen.

11. Stent nach Anspruch 1, umfassend Expansionsstreben (54) mit einem erhöhten Abschnitt an einem proximalen Ende und einem vertieften Abschnitt (58) an einem distalen Ende.

12. Stent nach Anspruch 1, umfassend geradlinige Expansionsstreben.

13. Stent nach einem beliebigen der vorhergehenden Ansprüche, wobei jede treppenförmige Expansionsstrebe (54) ein proximales Ende, ein distales Ende und ein treppenförmiges Segment (56, 58), das entweder benachbart zum proximalen Ende oder zum distalen Ende ist, aufweist.

14. Stent nach einem beliebigen der vorhergehenden Ansprüche, wobei jede Strebenpaarschleife eine treppenförmige Expansionsstrebe mit einem treppenförmigen Absatz an einem distalen Ende und eine treppenförmige Expansionsstrebe mit einem treppenförmigen Absatz an einem proximalen Ende aufweist.

## Revendications

1. Extenseur dans un état non déployé, comprenant :
• une première colonne d'expansion (48) comprenant des entretoises d'expansion en marche d'escalier individuelles (54) formant une pluralité de boucles de paire d'entretoises d'expansion qui couplent des entretoises d'expansion individuelles (54) adjacentes, dans lequel deux boucles de paire d'entretoises d'expansion adjacentes partagent une entretoise d'expansion en marche d'escalier (54) commune ;
• une deuxième colonne d'expansion (48) comprenant des entretoises d'expansion en marche d'escalier individuelles (54) formant une pluralité de boucles de paire d'entretoises d'expansion qui couplent des entretoises d'expansion individuelles (54) adjacentes, dans lequel deux boucles de paire d'entretoises d'expansion adjacentes partagent une entretoise d'expansion en marche d'escalier (54) commune ;
• et une première colonne d'entretoises de connexion (32, 134) comprenant une pluralité d'entretoises de connexion individuelles à géométrie symétrique (92), dans lequel chaque extrémité d'une entretoise de connexion individuelle (92) se déploie de manière ipsilatérale depuis des côtés de boucles de paire d'entretoises d'expansion des première et deuxième colonnes d'expansion (48),
• **caractérisé en ce que** chaque boucle de paire d'expansion comprend une première entretoise d'expansion en marche d'escalier (54) et une deuxième entretoise d'expansion en marche d'escalier (54).

2. Extenseur selon la revendication 1, dans lequel chaque entretoise de connexion (92) de la première colonne d'entretoises de connexion (32, 134) comprend des tiges courtes bilatérales (100, 102) qui sont conjointes de manière ipsilatérale à une entretoise d'expansion (54) de la première colonne d'expansion (48) et à une entretoise d'expansion (54) de la deuxième colonne d'expansion (48).

3. Extenseur selon la revendication 1, dans lequel au moins une entretoise de connexion (92) de la première colonne d'entretoises de connexion (32, 134) présente une configuration géométrique asymétrique.

4. Extenseur selon la revendication 1, dans lequel chaque entretoise de connexion (92) de la première colonne d'entretoises de connexion (32, 134) présente au moins six rayons de courbure.

5. Extenseur selon la revendication 1, dans lequel des boucles de paire d'entretoises d'expansion des première et deuxième colonnes d'expansion (48) sont alignées dans une géométrie vallée-crête.

6. Extenseur selon la revendication 1, dans lequel les boucles de paire d'entretoises d'expansion des première et deuxième colonnes d'expansion (48) sont alignées dans une géométrie crête-crête.

7. Extenseur selon la revendication 1, dans lequel la section centrale d'au moins une portion des entretoises de connexion (92) dans la première colonne d'entretoises de connexion (32, 134) s'étend dans une première direction et la section centrale d'au moins une portion des entretoises de connexion (92) dans la première colonne d'entretoises de connexion (32, 134) s'étend dans une deuxième direction.

8. Extenseur selon la revendication 1, **caractérisé en ce que** les entretoises d'expansion en marche d'escalier (54) comprennent une première section, une deuxième section et une troisième section disposée entre les première et deuxième sections, les première et deuxième sections étant parallèles l'une à l'autre, dans lequel la troisième section n'est pas parallèle à la première section et à la deuxième section.

9. Extenseur selon la revendication 1, dans lequel quelques-unes des entretoises d'expansion en marche d'escalier (54) présentent une section relevée à une extrémité proximale.

10. Extenseur selon la revendication 1, dans lequel quelques-unes des entretoises d'expansion en marche d'escalier (54) présentent une section relevée à une extrémité distale.

11. Extenseur selon la revendication 1, comprenant des entretoises d'expansion (54) avec une section relevée à une extrémité proximale et une section abaissée (58) à une extrémité distale.

12. Extenseur selon la revendication 1 comprenant des entretoises d'expansion rectilignes.

13. Extenseur selon l'une quelconque des revendications précédentes, dans lequel chaque entretoise d'expansion en marche d'escalier (54) comprend une extrémité proximale, une extrémité distale, et un segment en marche d'escalier (56, 58) positionné de manière adjacente soit à l'extrémité proximale, soit à l'extrémité distale.

14. Extenseur selon l'une quelconque des revendications précédentes, dans lequel chaque boucle de paire d'entretoises comprend une entretoise d'expansion en marche d'escalier avec une marche d'escalier à une extrémité distale et une entretoise d'expansion en marche d'escalier avec une marche d'escalier à une extrémité proximale.
